# EUROPEAN PATENT APPLICATION

(11) **EP 3 628 259 A1**
(43) Date of publication of application: **01.04.2020**
(21) Application number: 18315028.3
(22) Date of filing: 28.09.2018
(51) Int. Cl.: A61B 34/00

(54) **MEDICAL DEVICE AND METHOD FOR PERFORMING A SURGICAL OPERATION IN A BODY**

(71) Applicant: Artedrone, 75008 Paris (FR)
(72) Inventor: POULETTY, Philippe, 75005 Paris (FR)
(74) Representative: Hepp Wenger Ryffel AG

(57) **Abstract**

The present invention relates to a medical device (10), preferably a micro robot for application inside a body, preferably for application inside a human body (2). The medical device (10) includes a body part (11) and a tail part (12). A recapture line (13) is attached to the tail part (12). The recapture line (13) has a tensile strength sufficient to pull back the device and column strength not sufficient to push the medical device (10).

## Description

The present invention relates to a medical device and a method for performing a surgical operation in a body. In some non-limiting examples the medical device relates to a micro robot for application inside a human body.

Minimally invasive procedures, also known as minimally invasive surgeries, are surgical techniques that only require minimal size incisions and therefore require less wound healing time and reduce the risk of trauma in a patient. Specific tools were designed for minimally invasive surgeries such as catheters, fibre optic cables, grippers and pincers on long sticks or miniature video cameras.

A limitation of minimally invasive surgeries is that the surgeon may have to use tools that require a calm hand, which can be exhausting for long operations.

A further development in the field of minimally invasive surgery is robot-assisted surgery or robotic surgery. Hereby robotic systems are used in surgical procedures to assist the surgeon. Multiple robotic arms may perform a minimally invasive surgery while the surgeon handles the robotic arms e.g. with joysticks. However the operation is still invasive to some extent and creates internal and external wounds, which require healing time.

An even further development are micro robots that are injected into the human body to perform a diagnosis, a surgery or a treatment. These micro robots can be used for diagnosis or monitoring a disease in real time measuring glucose levels in diabetic patients or for delivering drugs to a targeted location, for example a tumor (Ornes, 2017, PNAS). These micro robots are small devices and have a size ranging from a few millimetres to a few microns.

Edd et al. have disclosed a surgical micro-robot that swims inside the human ureter and is proposed to provide a novel method of kidney stone destruction (Proceedings 2003 IEEE). Peyer et al. disclosed a swimming micro robot with artificial bacterial flagella to navigate in fluids of different viscosities (2012 IEEE). Micro robots are unable to carry batteries and motors due to their size. A popular way to guide the micro robots to the target locations is to control a micro robot comprising magnetic materials using external magnetic fields. The Multi-Scale Robotic lab at ETH Zurich disclosed an untethered micro robot with a diameter of 285 µm to perform eye surgeries.

The small size of these micro robots limits their ability to move against a fluid stream such as the blood stream. Magnetic fields can guide or stop the robot, but might not be strong enough to move the robot against a blood stream flowing in an opposite direction.

The invention seeks to mitigate one or more of the above issues, in particular to provide a medical device, preferably a micro robot, that is simple to produce and to use. Some embodiments have the additional advantage of being reliable and enabling a save recapture.

According to the invention the problem is solved with the characterising features of the independent claims.

The invention relates to a medical device. The medical device may be a micro robot for use in a body vessel. In particular, the medical device or micro robot may be suitable for application inside a human body. The medical device includes a body part and a tail part. A recapture line is attached to the device, preferably to the tail part and may be adapted to pull back the medical device from a first position. In one embodiment, the recapture line may have a stiffness not sufficient to move the medical device to a target location.

The first position may in particular be an target site of the medical device.

The recapture line may have a tensile strength sufficient to pull back the medical device and may have a column strength not sufficient to push the medical device against a force created by static or dynamic body fluids. Therefore, the line can be formed sufficiently thin such as to be easily insertable into a body duct.

As used herein the term "line" is intended to cover any construction to fulfil the task of pulling the device while optionally being able to fulfil other non-limiting tasks as well.

The medical device may be adapted to be injected into a body, in particular a human body. The tail part and optionally the body part may have a larger cross-section than the recapture line. The medical device may be pulled back mechanically or manually. Such a recapture line allows pulling the medical device through an opposing fluid stream, for example a blood stream. This pulling movement can be a slight adjustment of the position or a recapture of the medical device. In particular, the device may comprise a handle for the recapture line.

The recapture line may have a length configured to extend from the medical device to an insertion site of the medical device. One embodiment of the invention relates to a system comprising a port and a medical device wherein the recapture line extends from the tail part to the port.

The recapture line may be a string, in particular a flexible string. Advantageously, the string is bendable. An advantage is that such medical devices can be small in size and only requires a small incision, as compared to the known catheter devices.

The medical device can be released into a body vessel, carried through the body vessel by a fluid flow to a target site in a vessel, and recaptured in a simple manner. The device may be repositioned by loosening the recapture line or pulling the recapture line.

The medical device has preferably at least one drive for actively moving the device in a direction and a control member for controlling and preferably changing the movement of the medical device inside the body. The medical device can move within a body fluid flow and/or moving on a tissue.

The drive can be any kind of functionality that moves the medical device. Possible embodiments could be a propeller, wheels, caterpillar, flagellum, legs, hooks or a magnetic drive for external steering. The control member can move or steer or stop the device by external effects e.g. signals. The control member can adjust the speed or rotation direction of the drive and thereby control the position. The drive may allow the medical device to navigate through sharp turns in the blood vessels.

The medical device has preferably a positioning means to determine the position of the medical device in the body. The positioning means emits a signal, which is received by a receiver. The receiver than calculates the position of the medical device. This signal could be a radio wave, radioactive tracer, sound wave, Bluetooth, or any other wireless signal. In an alternative embodiment the positioning means could include sensors for measuring different environmental parameters such as temperature, pH, redox potential, salt concentration, viscosity, pressure, electric potential, gas concentration, radioactivity and or metabolic levels. The positioning means sends the measured parameters to the receiver, which calculates the position of the medical device. The measured parameters could also be used to analyse the environment.

The recapture line of the medical device preferably comprises a transmission cable, to transmit energy and/or data, in particular light or electric signals from or to the medical device. The transmission cable could include two separate cables, one for delivery energy and data and one for receiving data. The transmission cable could also be a single cable to transmit energy and data and figure as a recapture line.

The recapture line may comprise or consist of a biocompatible material. The recapture line preferably comprises or substantially consists of a material of the group of materials consisting of: metals, in particular copper, polymers, carbon fibres, nylon, polymers, silk, and carbon nanotubes, in particular graphene.

These materials are biocompatible and have a sufficient longitudinal strength for pulling the medical device. Further, the above materials resist degradation, preferably at least for a few hours or days and may degrade later in case the device is lost. This ensures that the medical device can be removed at any time, if necessary. Additionally, the materials resist environmental impact in the body, such as different pH or oxidative stress.

The medical device preferably comprises a material that is detectable by imaging techniques e.g. by MRI, CT scanner, echography, X-ray or fluoroscopy.

Thereby, the position of the device can be determined at any time during the procedure. If necessary, the positon can also be tracked, in particular in real time. A continuous localization process is beneficial, since the guiding of the medical device can be complicated depending on parameters such as viscosity of the fluid or external pressure from a body fluid stream.

The medical device may be particularly suited for blood vessels, in particular arteries or veins. Other areas of application may be the urethra or the ureter. The recapture line of the medical device has preferably an outer diameter of 10 to 1000 µm and more preferably of 50 - 200 µm.

The body part may comprise a magnetic part. This magnetic part is usable to guide the medical device by interaction with an external magnetic field. The magnetic part may be an inner core made of a magnetic material or comprising a magnetic material, magnetic micro- or nanoparticles in a matrix or a coating.

The medical device comprises preferably at least one functional unit such as a clamp, scalpel, drill, hook, stent, legs, caterpillar, propeller, detonator, camera or a sensor or a drug release component.

The functional unit can be attachable to the medical device. The functional unit can be used to move the medical device on a tissue or through a fluid. It can also be used to attach the medical device to a tissue site or to open a passage through a blocked opening or to create a new opening. Alternatively the functional unit can be used to collect data from the body environment.

The proposed device is particularly suitable to remove thrombosis in arteries, fill aneurysms or deliver drugs to a tumor. The detonator could open a thrombus.

The functional unit may activatable. In some embodiments the functional unit is activated with a magnetic field or electromagnetic waves in certain embodiments. This allows e.g. controlled release of a drug. The functional unit may be activatable by energy, e.g. electrical signals. The line

The medical device comprises preferably a reservoir to store and release a drug. The reservoir can be used to apply drugs to specific application sites. For example tumor cells can be locally treated with a toxic drug. The medical device is thereby used to transport the toxic drug to the application site and release it there. The controlled release of drugs enables also the possibility of timed drug application. The medical device can be inserted, guided to the site of application and wait until the scheduled release time of the drug. It is also possible to control the delayed release of two different drugs for example an active drug and an enzyme to deactivate the drug.

The medical device comprises preferably a transmitter to send data from the medical device to a receiver, particularly through the recapture line.

The recapture line may be adapted to transmit energy. Thereby, data obtained by a sensor in the medical device may be transmitted.

The device could be adapted to receive energy trough the recapture line and/or send data obtained by sensors in the medical device, in particular in the body part, through the recapture line. In additional or alternative embodiments, the medical device or the recapture line may comprise a wireless transmitter and/or a wireless receiver for sending and/or receiving energy or data.

The medical device has preferably a size of 8 - 2000 µm, preferably 50 - 1000 µm and more preferably 200 - 500 µm. The size may be a length, a diameter, or a longest dimension of the medical device.

The body and/or tail part of the medical device preferably comprise a material such as metal, plastic, glass, mineral, ceramic, carbohydrate, nitinol, carbon, biomaterial, or a biodegradable material.

The invention further provides a method for performing a surgical operation in a body, preferably a human body. In a first step a medical device is inserted into a body. The medical device is then navigated to a place of interaction, without pushing a recapture line. In particular, the medical device is inserted upstream of a target site. A fluid stream may carry the medical device to the target site. The medical device may be positioned by loosening or pulling the recapture line.

The medical device may perform one or several actions at one or several places. The medical device is removed from the body by pulling on the recapture line.

The invention further provides a system for controlling a medical device. The system comprises a medical device, preferably a medical device as described before and a magnetic field generator. The medical device is then guided by a magnetic field generated by the magnetic field generator.

The external magnetic field generator creates a magnetic field with a gradient of 0.1 to 20 T/m, preferably of 0.2 - 1 T/m. Once the medical device is inserted into the body, the magnetic field can be used to guide the medical device to the application site. Therefore the medical device is moved or stopped or steered by the magnetic field, in particular while floating in a body fluid stream. During the entire time the medical device remains attached to the recapture line.

In further embodiments, the medical device may have a magnetic anisotropy. Thereby, the medical device can be oriented by the magnetic field.

The invention further refers to a medical device, preferably a micro robot for application inside a body, preferably a human body.

Non-limiting embodiments of the invention are described, by way of example only, with respect to the accompanying drawings, in which:
- Fig. 1:: Schematic view of a medical device.
- Fig. 2:: Schematic view of an insertion site of a human body for the medical device.
- Fig. 3:: Schematic view of the medical device with a drive and a control member.
- Fig. 4:: Schematic view of the medical device with a positioning means.
- Fig. 5:: Schematic view of pulling the medical device with a magnetic field.
- Fig. 6:: Schematic view of data and energy transmission through a recapture line of the medical device.
- Fig. 7a-d:: Schematic view of functional units attached to the medical device.
- Fig. 8:: Schematic view of a tumor and antibodies delivered to the tumor by the medical device.

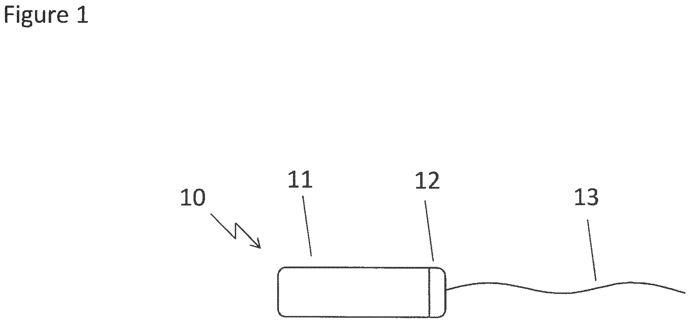

Figure 1 shows a schematic view of a medical device 10 comprising a body part 11 and a tail part 12. A recapture line 13 is attached to the body part 12. The recapture line 13 is used to pull the medical device 10.

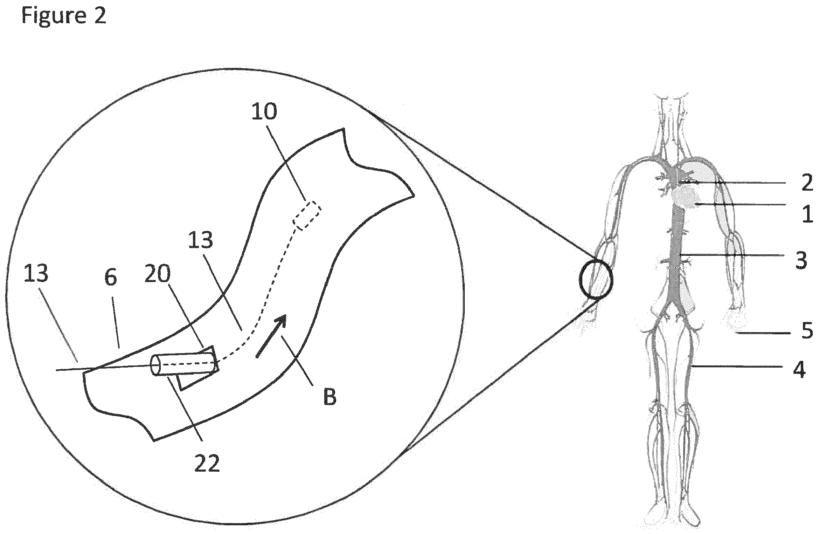

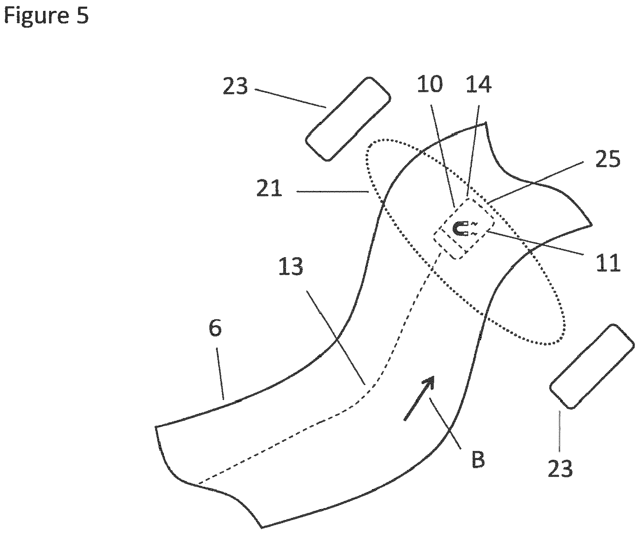

Figure 2 shows a schematic view of an insertion site 20 of a human body 2 for the medical device 10. The heart 1 is connected to a bloodstream. The blood stream comprises different types of blood vessels 6 such as aorta 3, veins 4 and capillaries 5. The medical device 10 is inserted into the blood vessel 6 at the insertion site 20. Therefore the blood vessel 6 is perforated by a catheter 22 at the insertion site 20. The medical device 10 is inserted into a blood stream B. The blood stream B is carrying the medical device 10 through the blood vessel until the medical device reaches a site of interaction 25 (Figure 5). At any time the medical device 10 is connected to the recapture line 13 and can be pulled back to the site of insertion 20.

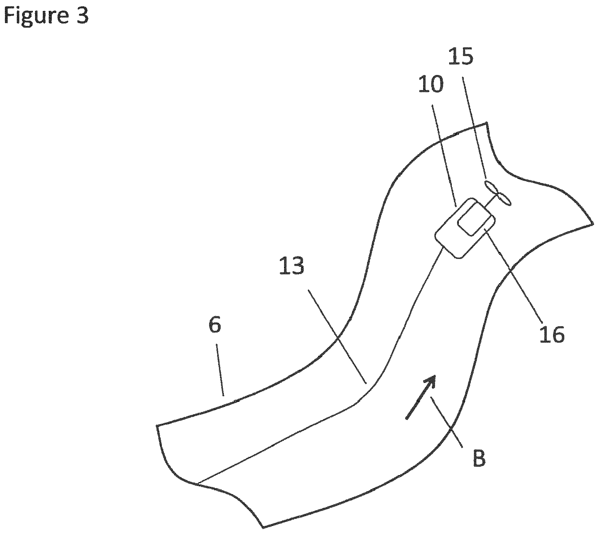

Figure 3 shows the medical device 10 with the recapture line 13 in a blood vessel 6. The medical device 10 has a drive 15 and a control member 16, to control the drive. The drive 15 actively moves the medical device 10 in a direction. The control member 16 modifies the action of the drive 15. The control member 16 can invert the rotation direction of the drive 15 or adjust its speed.

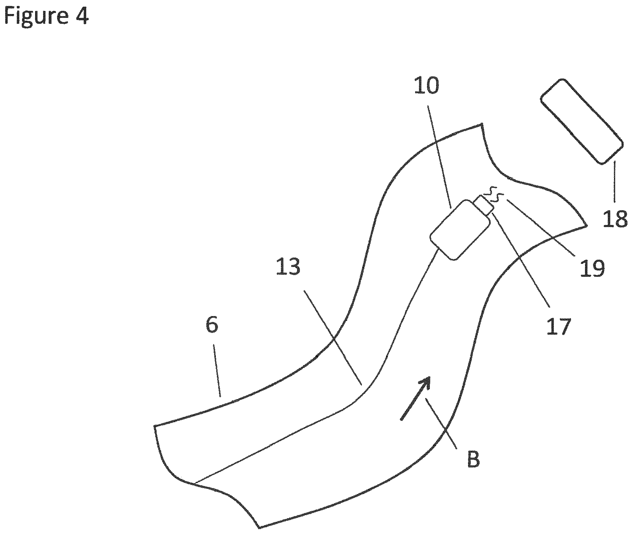

Figure 4 shows the medical device 10 with the recapture line 13 in a blood vessel 6. The medical device 10 has a positioning means 17. The positioning means 17 emits a signal 19, which is received by a receiver 18. Based on the signal 19, the receiver 18 calculates the position of the medical device 10.

Figure 5 shows a schematic view of the blood vessel 6 with the medical device 10. The medical device 10 is transported by the blood stream B and attached to the recapture line 13. A magnetic field generator 23 is generating a magnetic field 21 at the application site 25. The body part 11 of the medical device 10 has a magnetic part 14, which is attracted by the magnetic field 21. At the application site 25 the medical device 10 stays in place, held by the magnetic field 21 against force of the blood stream B. After performing any kind of action the magnetic field generator 23 is switched off and the magnetic field 21 collapses. The medical device is removed against the force of the blood stream B by pulling at the recapture line 13.

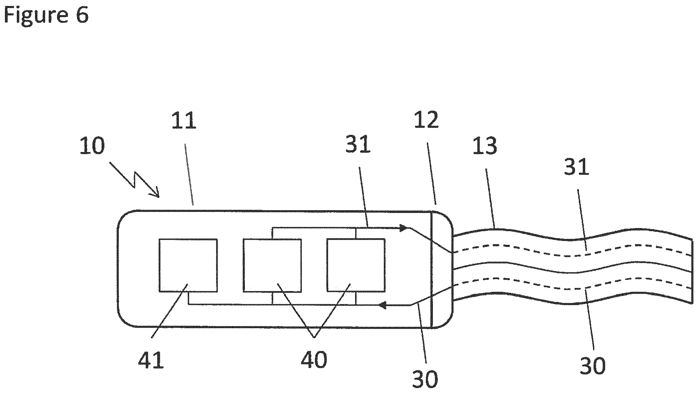

Figure 6 shows a schematic view of the medical device 10. The recapture line 13 comprises an energy transmission cable 30 and a data transmission cable 31. The energy transmission cable 30 transmits energy to sensors 40 and a compartment 41. The sensors send data through the data transmission cable 31. As an alternative the energy transmission cable 30 and the data transmission cable 31 can be integrated into the same cable. This cable is used to transport energy to and data to and from the medical device through the recapture line 13.

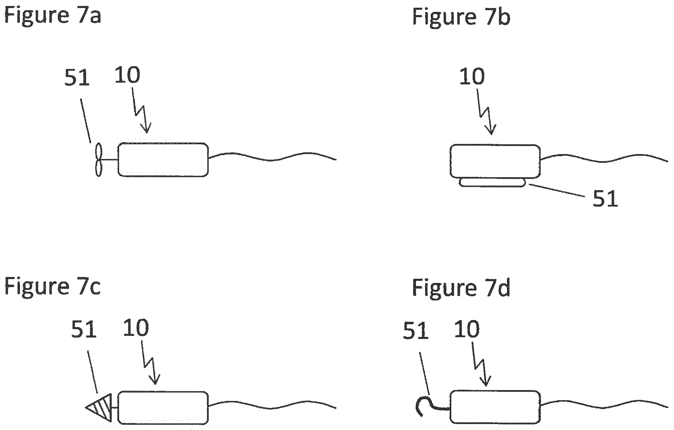

Figure 7a-d shows a schematic view of the medical device 10 with attachable functional units 51. In Figure 7a the functional unit 51 is a propeller to move the medical device 10 in a forward or reverse direction along a longitudinal axis through the device. Figure 7b shows a medical device 10 where the functional unit 51 is a caterpillar. The caterpillar is used to move the medical device 10 onto a tissue site. In figure 7c the functional unit 51 of the medical device 10 is a drill. The drill can be used to perforate a tissue and creating an opening to move across physical barriers. In Figure 7d the functional unit 51 of the medical device 10 is a hook. The hook can be used to hold the medical device 10 in place or to drag an object or material, when the medical device 10 is recaptured.

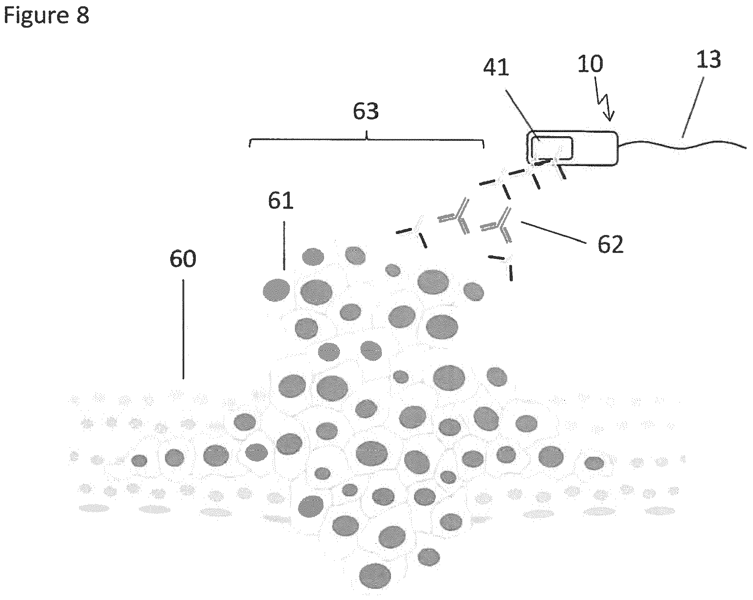

Figure 8 shows a schematic view of a tumor site 63. The tumor cells 61 have a bigger size and a faster replication cycle than the normal cells 60. The medical device 10 is guided to the tumor site and carries tumor specific antibodies 62 in the compartment 41. At the tumor site 63 the medical device 10 releases the tumor specific antibodies 62. The antibodies bind to the tumor cells and induce an immunotherapeutic process. After releasing the antibodies 62 the medical device 10 is removed from the tumor site 63 by pulling on the recapture line 13.

## Claims

1. A medical device (10), preferably a micro robot for use in a body vessel, preferably for application inside a human body (2), said medical device (10) including:
a body part (11) and
a tail part (12), **characterized in that** a recapture line (13) is attached to the device, preferably the tail part (12), wherein the recapture line (13) is adapted to pull back the medical device (10) from a target location and a stiffness not sufficient to move the medical device (10) to a target location.

2. The medical device (10) as claimed in claim 1, **characterized in that** the medical device (10) has at least one of:
i) a drive (15) for actively moving the device in a direction.
ii) and a control member (16) for changing the movement direction within a body by external effects.

3. The medical device (10) as claimed in one of the claims 1 or 2, **characterized in that** the medical device (10) has a positioning means (18) to determine the position of the medical device (10) in the body.

4. The medical device (10) as claimed in one of the claims 1 to 3, **characterized in that** the recapture line (13) comprises a transmission cable (30, 31) to transmit energy and/or data, in particular light or electric signals to the medical device (10).

5. The medical device (10) as claimed in one of the claims 1 to 4, **characterized in that** the recapture line (13) comprises a material of the group of materials consisting of metals, in particular copper, polymers, carbon fibres, graphene, a fabric, silk, protein fibres and carbon nanotubes.

6. The medical device (10) as claimed in one of the claims 1 to 5, **characterized in that** the recapture line has a smaller cross-section than the medical device.

7. The medical device (10) as claimed in one of the claims 1 to 6, **characterized in that** the medical device (10) comprises a material that enables detection by at least one of a group of imaging techniques comprising: IRM, scanner, echography, X-ray, fluoroscopy.

8. The medical device (10) as claimed in one of the claims 1 or 7, **characterized in that** the recapture line (13) has an outer diameter of 10 to 1000 µm , preferably 50 µm to 200 µm.

9. The medical device (10) as claimed in one of the claims 1 to 8, **characterized in that** the body part (11) contains a magnetic part (14) .

10. The medical device (10) as claimed in one of the claims 1 to 9, **characterized in that** the body part (11) contains at least one functional unit (51), wherein the functional unit (51) is in particular selected from a group consisting of: clamp, scalpel, drill, hook, stent, legs, caterpillar, propeller, detonator, camera and sensor.

11. The medical device (10) as claimed in one of the claims 1 to 10, **characterized in that** the body part (11) comprises a compartment (41) configured to store and release a drug (62).

12. The medical device (10) as claimed in one of the claims 1 to 11, **characterized in that** the body part (11) contains a transmitter (17) configured to send data from the medical device to a receiver (18), in particular through the recapture line (13).

13. The medical device (10) as claimed in one of the claims 1 to 12, **characterized in that** the medical device has a size of 8 - 2000 µm, preferably 50 - 1000 µm and more preferably 200 - 500 µm.

14. The medical device (10) as claimed in one of the claims 1 to 13, **characterized in that** the body part (11) and tail part (12) of medical device (10) comprise a material selected from the group of: metal, plastic, glass, mineral, ceramic, carbohydrate, nitinol, carbon, biomaterial, or a biodegradable material.

15. A method for controlling a medical device, preferably a human body (2), comprising the following steps of:
- Insertion of the medical device (10), preferably a medical device (10) according to one of the claims 1 to 14 into a body at an insertion site, wherein the medical device includes a body part (11) and a tail part (12);
- Navigating the medical device (10) to a target site (25) without pushing a recapture line (13), wherein the recapture line is preferably attached to the tail part (12) of the medical device (10);
- Removing the medical device (10) from the target site (25), by pulling the recapture line (13).

16. A system for controlling a medical device (10) comprising a medical device (10) according to claim 9 and a magnetic field generator (23) **characterized in that**, the medical device (10) is guidable by a magnetic field (21) generated by the magnetic field generator (23).
